(19) Europäisches Patentamt
European Patent Office
Office européen des brevets

(11) Publication number: 0 306 167 A2

(12) **EUROPEAN PATENT APPLICATION**

(21) Application number: 88307507.9

(22) Date of filing: 12.08.88

(51) Int. Cl.4: C12N 9/36 , C12N 9/12 , C12N 9/88

(30) Priority: 03.09.87 US 92724

(43) Date of publication of application: 08.03.89 Bulletin 89/10

(84) Designated Contracting States: BE CH DE ES FR GB IT LI NL SE

(71) Applicant: TECHNICON INSTRUMENTS CORPORATION
511 Benedict Avenue
Tarrytown, New York 10591(US)

(72) Inventor: Saini, Mohan S.
27 Harmony Road
Spring Valley New York 10977(US)

(74) Representative: Wain, Christopher Paul et al
A.A. THORNTON & CO. Northumberland House 303-306 High Holborn
London WC1V 7LE(GB)

(54) **Stabilization of enzymes by inhibiting proteolytic enzyme contaminants.**

(57) Aqueous solutions of enzymes are stabilized with proteolytic inhibitors. For example, phosphoenol pyruvate carboxylase is stabilized with phenyl methyl sulfonyl chloride, α-antitrypsin inhibitor, partially purified ovomucoid, ovoinhibitor or soya bean trypsin factor; and a mixture of phosphoglucomutase and N-acetyl phosphoglucoseamine mutase is stabilised with soya bean trypsin inhibitor, antitrypsin inhibitor, aprotinin, p-aminobenzamidine, leupeptin, benzamidine or α,α′-bipyridal.

EP 0 306 167 A2

Xerox Copy Centre

# STABILIZATION OF ENZYMES BY INHIBITING PROTEOLYTIC ENZYME CONTAMINANTS

The present invention relates to enzyme stabilization and, in particular, the use of proteolytic inhibitors to stabilize non-proteolytic enzymes, eg. phosphoenol pyruvate carboxylase, and a phosphoglucomutase and N-acetyl phosphoglucoseamine mutase enzyme solution.

Phosphoenol pyruvate carboxylase (PEPC) is used in automatic analyzers for determining the amount of carbon dioxide in blood. The amount of carbon dioxide in the blood stream (or other body fluids) is an important measurement in medical diagnostics. Abnormal concentrations of carbon dioxide in the blood stream are either the product of or, in some circumstances, the cause of a variety of illnesses: metabolic and respiratory acidosis and alkalosis.

Only a small portion of the carbon dioxide introduced into the bloodstream remains in the physically dissolved state. It may be dissolved in the erythrocytic cells (red blood cells) of the blood in which the dissolved carbon dioxide is converted into carbonic acid by the catalytic action of carbonic anhydrase. The carbonic acid in turn dissociates into hydrogen ions and bicarbonate ions ($HCO_3^-$) at the pH of blood. In order to restore the equilibrium, $HCO_3^-$ diffuses out of the cells into the plasma. The determination of the amount of $HCO_3^-$ present by spectrophotometry can be achieved by coupling $HCO_3^-$ to a material(s) which exhibit(s) light absorption. The amount of this material present is determined photometrically and the concentration of bicarbonate originally present is thus determined. Phosphoenol pyruvate carboxylase (PEPC) is used for this purpose, and the reaction involved is:

$$\text{phosphoenol pyruvate} + HCO_3^- \xrightarrow{\text{PEPC}} \text{oxaloacetate} + P_1$$

where $P_1$ is inorganic phosphate. The thus-formed oxaloacetate is further reacted:

$$\text{Oxaloacetate} + \text{NADH} \xrightarrow{\text{MDH}} \text{malate} + \text{NAD}$$

where MDH is malate dehydrogenase, and NADH and NAD, respectively, are the reduced and oxidized forms of nicotinamide adenine dinucleotide. Either the rate of conversion of NADH to NAD or the end point of the reaction can be measured following standard protocols.

The rate process uses substantially less enzyme than the end point process and in view of the high cost of the enzyme, it is preferred. However, one problem involved in performing the $HCO_3^-$ determination by either of the above methods is that PEPC tends to lose its activity on storage thereby causing erroneous determinations.

One known method for the stabilization of phosphoenol pyruvate carboxylase is described in U. S. Patent No. 3,963,578. PEPC reportedly is stabilized to have a shelf life of six months or more at 4° C by incorporating the phosphenol pyruvate carboxylase in an aqueous solution containing from 0.002 to 0.05 molar sodium phosphate, from 1 to 10, and preferably 3 to 8, m molar aspartate, from 0.02 to 0.1 m molar ethylene diamine tetracetic acid, from 0.6 to 3.15, and preferably 0.8 to 1.2, molar ammonium sulfate and up to 20 volume percent glycerol. The sodium phosphate acts as a buffer to maintain the pH of the solution at from 6.0 to 6.8. However, this patent gives no indication of the cause of the instability of the enzyme. The patent is directed to stabilizing the enzyme at a storage temperature of about 4° C. There is no discussion of nor are any examples given of the stability of the disclosed enzyme solution at an elevated temperature, i.e. - at ambient or a higher temperature at which the typical reaction described above occurs. In addition, the high salt and buffer concentrations in the solution would restrict utilization of the solution in automatic clinical analyzers and often may not be useful for stabilizing the activity of other enzymes.

The enzymes phosphoglucomutase (PGM) and n-acetyl phosphoglucoseamine mutase (PAGM) are attractive for use in enzyme-linked immunoassays. PGM has a high potential for use as an indicator enzyme because: (a) it (diphospho form) has a very strong affinity for glucose-1,6-disphosphate (Km, $10^{-8}$M; W. Ray and E. Peck, The Enzymes, 6, 407, 1972); (b) the specific activity of PGM, i.e., 500 (V. Deshpande and J. Joshi, J. Biol. Chem, 260,757 1985) corresponds to a turnover number of 32,500. Glucose-1,6-disphosphate is both a substrate and an activator for PGM and PAGM (see below). This means that each active molecule of PGM will give rise to 32,500 molecules of glucose-6-phosphate when glucose-1-phosphate is allowed to react with phosphoenzyme (active form of PGM). Production of glucose-6-phosphate can be quantified in the presence of glucose-6-phosphate dehydrogenase and NAD. NAD is reduced to NADH. The later has λ max at 340 nm (W. Ray and G. Roselli, J. Biol. Chem. 239, 1228, 1964). Both of these properties make PGM a useful enzyme for measuring analytes of lower concentration by biochemical and immunochemical methods.

N-acetyl phosphoglucoseamine mutase (PAGM) can react on the derivatives of glucose-1,6-diphosphate. (A. Sorenson and D. Carlson, J. Biol. Chem. 246, 3485, 1971). Thus, the serum analytes linked to the amino group of 2-amino glucose-1,6-diphosphate should be able to phosphorylate (activate) PAGM. The phospho form of PAGM can give rise to glucose-1,6-disphosphate. Glucose-1,6-diphosphate is better utilized by PGM than PAGM.

In an immunochemical assay, antibody can be bound either by free analyte or analyte conjugated to glucose-1,6-disphosphate. The other potential use of these enzymes is in the areas where serum constituents of clinical interest affect the activity of these enzymes directly.

We have now found that the useful life of certain enzymes can be extended, so that accurate clinical determinations utilizing such enzymes as reagents can be obtained. In particular, we have found that aqueous enzyme compositions can be stabilised using proteolytic inhibitors.

Thus ,the invention provides an aqueous enzyme composition which comprises a non-proteolytic enzyme and a proteolytic enzyme inhibitor.

It is known that phosphoenol pyruvate carboxylase can be isolated from any of Escherichia coli, all strains including strain B and strain W, Salmonella typhinurium, Brevibacterium flavin, which are essentially identical for this purpose, Bacillus stearothermophillus, Arthrobacter globiformis, which are similar for this purpose, as well as other bacteria such as Acetobacter xylinum. Undoubtedly other bacteria can also be used.

The relative instability of phosphoenol pyruvate carboxylase is known. Through experimentation, we have found that a possible cause of this instability is the presence of proteases, which degrade protein. Further experimentation has shown us that phosphoenol pyruvate carboxylase can be stabilized by the addition of proteolytic inhibitors. Our experiments are summarized below.

First, the cause of the instability of the enzyme was determined. We made tests for the presence of proteases in enzyme preparations in commercially available sources, and these tests gave positive results, indicating proteolytic contamination and degradation of the enzyme.

Both organic and naturally occurring inhibitors were tested to suppress the activity of the proteases. Among the several inhibitors tested, phenyl methyl sulfonyl fluoride (PMSF),α-antitrypsin inhibitor, partially purified ovomucoid and ovoinhibitor in the presence of low amount of ammonium sulfate and soya bean trypsin inhibitor were found especially effective in stabilizing phosphoenol pyruvate carboxylase.

Crude soya bean trypsin inhibitor is the preferred stabilizer as it was found to protect the enzyme activity for five days and the solution remained clear on storage. This inhibitor is also less expensive than α-antitrypsin inhibitor and ovoinhibitor.

α-antitrypsin and ovoinhibitor are rather costly to use for product formulation. Partially purified ovomucoid is less expensive and is as effective as soya bean inhibitors α-antitrypsin and ovoinhibitor, but the solution becomes turbid on storage in the presence of ovomucoid. PMSF is the least desirable due to its toxicity.

While the enzyme activity of phosphoglucomutase (PGM) and n-acetyl phosphoglucoseamine mutase (PAGM) is present in microbes, animals and plants, the instability was evaluated in the case of porcine submaxillary glands where the instability is very severe. The enzymic activity of PGM and PAGM was found to drop by 50% over a 24 hour period at 4°C, during the initial stages of purification. We have found this to be due to the presence of proteases in the enzyme solution. Porcine submaxillary glands contain serine proteases and kallikreins in addition to trypsin. The kallikreins are proenzymes and are activated by other proteolytic enzymes such as trypsin. In order to block the proteolysis of the PGM/PAGM enzyme solution, a series of experiments was conducted using various inhibitors.

It was found that some protection against proteolysis was afforded by α-antitrypsin inhibitor over longer periods of time at pH 7.5. At this pH, soya bean trypsin inhibitor protected the enzymes against denaturation though not as effectively as it did at pH 5.0. The enzyme was stable for 3 weeks in its presence, as compared to essentially no stability in the absence of any protease inhibitor. TPCK did not show any additional inactivation of the PGM/PAGM enzyme at a pH of 7.5 as it did at pH 5.0.

Aprotinin was found to be the most potent stabilizer, stabilizing the enzyme solution for over one month. Leupeptin was equally effective. The enzyme solution was stabilized by soya bean trypsin inhibitor for one week. Enzyme activity of the solution was protected for 14 days in the presence of p-aminobenzamidine and for 5 days with benzamidine. α,α′-bipyridal was the least effective of the stabilizers tested.

For suppressing proteolytic activity during the purification of the PGM/PAGM solution, p-aminobenzazmidine was found to be suitable. Large volumes of the enzyme solution are prepared in the isolation of PGM/PAGM enzyme. Use of other protease inhibitors such as soya bean trypsin inhibitor, leupeptin and aprotinin is less desirable because of their cost. Further, during the final stages of purification of PGM/PAGM, the use of proteins as inhibitors of proteolytic activities is inappropriate because protein contaminants are difficult to separate from the enzyme preparation; however, p-aminobenzamidine is a low molecular weight compound and as such can be easily removed from the enzyme solution by dialysis or by pressure filtration.

In the accompanying drawing, which is presented to further describe the invention and to assist in its understanding through clarification of its various aspects, Fig. 1 is a plot comparing the stabilization of a PGM/PAGM enzyme solution by various proteolytic inhibitors.

The following working examples describe experiments which were performed in developing the present invention. Standard commercially available reagent grade chemicals were used whenever possible. These working examples are to be considered illustrative of the present invention and should not be interpreted as limiting its scope.

The activity assays to determine the stability of phosphoenol pyruvate carboxylase were done on a TECHNICON RA-1000 clinical analyzer in accordance with established protocols for such analysis. (TECHNICON RA-1000 is a registered trademark of Technicon Instruments Corporation, Tarrytown, N.Y.) In accordance with such protocol, the enzyme solution, $R_2$, was placed in the sample cup. The other constituents of the assay mixture, $R_1$, were placed in the reagent boat. 10 µl of $R_2$ were mixed with 350 µl of $R_1$. The reaction was done at 37°C and there was a 30 second incubation period before the readings were taken. The reported activity remaining refers to the percentage of the activity determined when fresh solutions were made initially.

The presence of proteolytic activity in sample phosphoenol pyruvate carboxylase was determined by a kit method supplied by Bio-Rad (Bulletin 1111). This is a very sensitive method and detection limit is down to ng quantities of proteases. In order to account for the activation of proteolytic enzymes during storage, the solid samples were dissolved in buffer as described below and stored in $R_2$ at 37°C on the TECHNICON RA-1000 clinical analyzer for 6 hours.

The solid enzyme samples were dissolved in 50 mM Hepes, pH 7.0 containing 0.1% benzoic and .01% gentamicin ($R_2$). The stability study was done in $R_2$ containing about 20 mg/ml enzyme at room temperature. The ratio of soya bean trypsin inhibitor to phosphoenol pyruvate by weight mg/ml was 10:20. The specific activity of soya bean was 7000 BAEE inhibitor units/mg and that of phosphoenol pyruvate was one unit/mg. The components of the activity assay were in $R_1$ as NADH, $1.3 \times 10^{-4}$M; phosphoenol pyruvate, $2.5 \times 10^{-3}$M; magnesium acetate, $1.06 \times 10^{-2}$M and $NaHCO_3$, $5 \times 10^{-3}$M; were in 104 mM tris-acetate pH 8.3 (at room temperature).

The PEPC enzyme samples showed the presence of proteases. During the test for the presence of proteases, the samples were diluted and subjected to optimal conditions for the activity of proteolytic enzymes.

Having observed the presence of proteolytic enzymes as contaminants, several experiments were done to suppress their activity and to stabilize the phosphoenol pyruvate carboxylase. Inhibitors of proteolytic enzymes from a variety of sources were tested to achieve this goal.

Many of these inhibitors were against serine proteases as the latter are more likely to be active near neutral pH. The effect of some of these inhibitors on the stability of the enzyme is shown in Table I.

TABLE I

| STABILIZATION OF PEPC BY VARIOUS INHIBITORS | | | |
|---|---|---|---|
| Sample | Percent Activity Remaining at Time (hrs. | | |
| | 2.5 | 5 | 23 |
| 1. Control | 80 | 80 | 25 |
| 2. Benzamidine (4mM) | 83 | 83 | 32 |
| 3. p-Amino benzamidine (4mM) | 75 | 75 | 28 |
| 4. TPCK (0.5mM) | 74 | 68 | 28 |
| 5. Soya Bean Trypsin Inhibitor (10 mg/ml) | 83 | 84 | 31 |
| 6. Egg white Trypsin Inhibitor (purified ovomucoid) (10 mg/ml) | 80 | 70 | 29 |
| 7. BSA (20mg/ml) | 87 | 87 | 46 |
| 8. Aprotinin (0.5 mg/ml) | 82 | 83 | 34 |
| 9. PMSF (1 mM) | 87 | 89 | 56 |

In most cases the enzyme was stable up to five (5) hours and there was no appreciable loss of activity during this time period. Instability was observed only after 23 hours at room temperature.

PMSF is a known serine protease inhibitor and, as shown, was the most effective stabilizer.

The effect of several inhibitors, including PMSF, was examined both in the presence and absence of 50mM $(NH_4)_2SO_4$ and the results are shown in Table II.

TABLE II

| STABILIZING EFFECT OF VARIOUS INHIBITORS IN PRESENCE OF AMMONIUM SULFATE | | |
|---|---|---|
| Sample | Percent Activity Remaining at Time (Hrs.) | |
| | 20 | 25 |
| 1. Control | 35 | 26 |
| 2. PMSF (1mM) | 71 | 61 |
| 3. PMSF (1mM) + $(NH_4)_2SO_{4\ (50\ mM)}$ | 90 | 88 |
| 4. α-Antitrypsin Inhibitor (5 mg/ml) | 58 | 58 |
| 5. α-Antitrypsin Inhibitor + $(NH_4)_2SO_4$ | 79 | 82 |
| 6. p-Nitrophenyl-p-Guanidinobenzoate (0.1mg/ml) | 42 | 36 |
| 7. p-Nitrophenyl-p-Guanidinobenzoate + $(NH_4)_2SO_4$ | 60 | 57 |
| 8. Ovomucoid (10 mg/ml) | 64 | 63 |
| 9. Ovomucoid + $(NH_4)_2SO_4$ | 83 | 87 |
| 10. D-Tryptophan methyl ester (5mM) | 32 | 26 |
| 11. D-Tryptophan-methyl ester + $(NH_4)_2SO_4$ | 68 | 71 |
| 12. 4-Phenyl butylamine (10mM) | 57 | 51 |
| 13. D-Arginine (10mM) | 30 | 24 |
| 14. D-phenylalanine (5mM) | 57 | 51 |
| 15. α-Antitrypsin Inhibitor + D-Tryptophan methyl ester + D-phenylalanine | 62 | 62 |
| 16. (12) + $(NH_4)_2SO_4$ | 82 | 89 |

PMSF in combination with ammonium sulfate stabilized the enzyme to the extent of 88% of original activity for 24 hours. α-antitrypsin inhibitor had a wide spectrum of specificity including trypsin, chymotrypsin, elastase and collegenase. It represented 90% of serum protease inhibitory activity. α-antitrypsin inhibitor stabilized the enzyme (Sample 4) to the extent that 58% activity was retained after 25 hours and in combination with $(NH_4)_2SO_4$, (Sample 5) 82% of the original activity was retained.

p-nitrophenyl-p-guanidinobenzoate is known to be an excellent active site titrant of many serine proteases. D-Tryptophan methyl ester, D-phenlyalanine and 4-phenyl butylamine are known to inhibit chymotrypsin. Turbidity developed on storage in the case of D-Tryptophan methyl ester, D-phenylalanine

and p-nitro-p-guanidino benzoate. Inclusion of D-Tryptophan methyl ester and D-phenylalanine with α-antitrypsin inhibitor did not offer any additional advantage over α-antitrypsin alone.

Ovomucoids are glycoproteins and known protease inhibitors obtainable from avian egg white. The crude preparation of ovomucoids inhibits chymotrypsin in addition to trypsin. The enzyme was protected to the extent of 87% in the presence of ovomucoid and $(NH_4)_2SO_4$.

The concentration of ovomucoid required to stabilize the enzyme was optimized. There was maximal protection of enzymic activity with 8-10 mg/ml of ovomucoid. Higher concentration causes turbidity on storing overnight. The optimal concentration of $(NH_4)_2SO_4$ in the absence of any inhibitor was determined to be 75 mM. Higher concentration resulted in precipitation of proteins on storage.

An experiment was done to explore the nature of stabilizing effect of $(NH_4)_2SO_4$, whether it is due to the general ionic strength effect or to the specific effect of the salt ions. Samples containing 10 mg/ml of ovomucoid were clear when NaCl replaced $(NH_4)_2SO_4$ at different concentrations up to 200 mM. The stabilization by NaCl was negligible. Hence, partial stabilization by $(NH_4)_2SO_4$ appears to be due to ions of this salt and not due to general salt-effect.

The effect of substrate phosphoenol pyruvate on protecting the enzymic activity was studied. The enzyme was unstable in the presence of 5 mM phosphoenol pyruvate. The substrate present in combination with ovomucoid and $(NH_4)_2SO_4$ did not impart any additional beneficial effect. Purified ovomucoid of chicken and turkey showed no stabilizing effect. The crude ovomucoid also contains ovoinhibitor which might be inhibiting the proteolytic activity or the ovomucoid before purification from ovomucoid has a different structure. The native form of ovomucoid may be more effective before it has been purified. It is also useful to note that ovomucoid is effective in inhibiting bovine trypsin and α-chymotrypsin (M. Lasowski, Jr. and R. Sealock, The Enzymes, 3, 375-484, 1984, 3rd Edition, Academic Press).

Next, an experiment was done to check the versatility of ovomucoid (partially purified) in stabilizing phosphoenol pyruvate carboxylase obtained from other commercial sources, e.g., A, Calbiochem and B, Beckman in addition to C, from Biozyme and the results are reported in Table III.

The samples were stored at room temperature in 50 mM Hepes buffer pH 7.0 containing 0.1% benzoate acid .01% gentamycin. The amount of enzyme and ovomucoid was respectively 20 mg/ml and 10 mg/ml in the presence of 75 mM ammonium sulfate.

TABLE III

| EFFECT OF OVOMUCOID ON THE STABILITY OF PEPC FROM DIFFERENT SOURCES | |
|---|---|
| Source | % Activity Remaining (after 24 hours) |
| A. | |
| Control<br>+ ovomucoid | 28<br>85 |
| B. | |
| Control<br>+ ovomucoid | 29<br>85 |
| C. | |
| Control<br>+ ovomucoid | 70<br>91 |

The enzymes from Sources A and B showed significant loss of activity and were stabilized to the same extent by the inhibitor. The loss was reduced to about 15% in both cases during an intitial 24 hour period. In the case of the Source C enzyme, a loss of 30% activity occurred in the absence of inhibitor and when ovomucoid was added, this loss was reduced to less than 10%.

Though ovomucoid is effective for three (3) days, it causes the enzyme solution to be turbid on storage. Other inhibitors were tested. Soya bean trypsin inhibitor and ovoinhibitor were found to preserve enzyme activity. Ovoinhibitor at a concentration of 0.5 mg/ml and 0.1 mg/ml protected the enzymic activity over a 24 hour period respectively to the extent of 90% and 80%.

Soya bean trypsin inhibitor stabilized phosphoenol pyruvate carboxylase for a period of five (5) days in the presence or absence of $(NH_4)_2SO_4$. The solution was clear when the sample did not contain $(NH_4)_2SO_4$. The enzyme was stable in the presence of soya bean trypsin inhibitor for five (5) days.

In another experiment, soya bean trypsin inhibitor from several sources was tested. These sources included U.S. Biochemicals, Calbiochem, Fluka, Boehringer Mannheim and Biozyme: Soy I, Soy II and Soy III.

The inhibitor from U. S. Biochemical was found to be the most effective and the second best was from Calbiochem. Soya bean trypsin inhibitor from Fluka, Boehringer-Mannheim and Biozyme was good for two days, but its effectiveness from these sources was diminished when checked at a later date.

Based upon the above experimentation, it can be concluded that the proteolytic degradation of phosphoenol pyruvate carboxylase can be inhibited by PMSF, α-antitrypsin inhibitor, ovoinhibitor and partially purified ovomucoid (H. Lineweaver and C. Murray, J. Biol. Chem. 171, 565 (1947) and soya bean trypsin inhibitor. PMSF is toxic and special care is required to handle this compound. α-antitrypsin and ovoinhibitor are expensive and their use may be cost prohibitory. Partially purified ovomucoid is not expensive and is soluble in the presence of salt $(NH_4)_2SO_4$. The ovomucoid preparation may be more convenient to use for the stabilization of the enzyme. PEPC from Biozyme and Calbiochem loses 70% activity during storage and in the presence of ovomucoid (10 mg/ml) and $(NH_4)_2SO_4$ (75 mm), the loss of enzyme is reduced to about 15% or about 85% of enzymic activity is retained. The Beckman PEPC enzyme, though comparable stable, still shows a loss of 30% enzymic activity and this loss is reduced to about 10% when ovomucoid is present. Purified ovoinhibitor has similar stabilizing effect on the enzyme.

From this study, it is apparent that soya bean trypsin inhibitor is a stabilizer of choice. It is less expensive than others considered in the study and the solution was found to remain clear on storage. In the case of ovomucoid and ovoinhibitor, turbidity develops even in the presence of $(NH_4)_2SO_4$. No other inhibitor was found to be as effective as soya bean trypsin inhibitor for long term stability of phosphoenol pyruvate carboxylase. The enzyme was stable to the extent of 80%-90% of its original activity when left in reconstituted reagent for five (5) days at room temperature. The use of this inhibitor will result in lowering the amount of phosphoenol pyruvate carboxylase required and consequently the cost per assay will be reduced. A comparison of all effective inhibitors is presented in Table IV.

TABLE IV

| VARIOUS INHIBITORS FOR THE PROTECTION OF PEPC ACTIVITY | |
|---|---|
| | Effectiveness (hours) |
| α-antitrypsin | 24-48 |
| Ovoinhibitor | 24-48 |
| Ovomucoid | 48-72 |
| PMSF | 24-48 |
| Soya bean Trypsin Inhibitor | 140 |

The other enzymes, stabilized by the teachings of the present invention were phosphoglucomutase (PGM) and n-acetyl phosphoglucoseamine mutase (PAGM). The enzyme activity is present in microbes, animals and plants. Porcine submaxillary glands are a rich sourse of these enzymes (D. Carlson, Methods in Enzymology, 8, 179, 1966). These tissues, however, also contain significant protease activity (M. Lemon and H. Fritt, Biochem. Journal, 177, 159 1979), and the proteolytic enzymes destroy the activity of the enzymes. As described hereinbelow, these enzymes were stabilized using protease inhibitors.

The presence of proteases was detected pursuant to the protocol discussed above. The quantitative assay for protease activity was done with N-α - benzoyl-L-arginine-p-nitroanalide as a substrate by a published procedure (H. Fritz, I. Truntschold and E. Werle; "Methods of Enzymatic Analysis", Ed. H. Bergmeyer, Vol. II, Academic Press, N.4. 1974). The method consist of (1) 0.2M triethanolamine buffer (pH 7.8) with 20 mM $CaCl_2$, (2) trypsin standard in 1 mM HCl, (3) substrate solution, 2.2 mM in water. The sensitivity can be enhanced four-fold if the concentration of the substrate was increased five times. A clear solution of the substrate was obtained by placing it in a boiling water bath for 5 minutes. The reaction mixture consisted of a .675 ml buffer and 0.325 ml substrate solution. The rate was measured at 405 nm at 37°C on a DU 8 spectrophotometer from Beckman Instruments, Inc. It was possible to detect protease

activity equivalent to 1 mg/L trypsin when the standard trypsin was activity of 11000 u/mg with substrate N-α-benzoyl-L-arginine p-nitroanalide. The activity of the PGM/PAGM enzyme solution was determined using the following reaction mixture. $NAD^{+}$, $10^{-3}$M; glucose-1-phosphate, $2x10^{-4}$M; glucose-1,6-diphosphate, $10^{-5}$M; Mg Acetate, 2 x $10^{-3}$M; EDTA, 2 x $10^{-4}$M and 1 u/ml glucose-6-phosphate dehydrogenase in 50 mM imidazole acetate, pH 7.5. The reaction was started by adding an aliquot of the enzyme solution and the rate was measured 37° C at 340 nm on a DU-8 spectro- photometer. This method is essentially similar to the literature procedures (W. Ray and E. Peck, The Enzymes, 6, 407, 1972. H. Bergmeyer. "Methods of Enzymatic Analysis" Ed. H. Bergmeyer Vol. I, Academic Press, N.Y. 1974).

The instability of the PGM/PAGM enzyme solution was found to be due to the presence of proteases. Porcine submaxillary glands contain serine proteases and kallikreins in addition to trypsin. The kallikreins are proenzymes and are activated by other proteolytic enzymes. The presence of proteases in enzyme preparation degrades PGM/PAGM to the extent of 50% overnight stored at 4° C.

A series of experiments were done to determine the stabilizing effect of various inhibitors. In one experiment the enzyme preparation included was 30-70% ammonium sulfate fraction. The effects of soya bean trypsin inhibitor (10 mg/ml), tosyl phenyl chloromethyl ketone (TPCK) (2mM) and α-antitrypsin inhibitor (2 mg/ml) were studied at pH 5.0 and 7.5, in 50 mM imidazole acetate buffer; 0.2 mM EDTA, 10 mM magnesium acetate. The stability was done at two pH values as the formation and dissociation of the inhibitor complex with trypsin is pH dependent. As shown in Fig. 1, the PGM/PAGM enzyme solution is stable for over three weeks at 5° C in the presence of soya inhibitor at pH 5.0. TPCK destroyed the activity sooner than the control. PGM had an active serine residue. PAGM enzyme may also have serine residues at the active site. The enzymes were rendered inactive in the presence of TPCK. There was some protection afforded by α-antitrypsin inhibitor over longer periods of time at pH 7.5. Soya bean trypsin inhibitor also protected the enzymes against denaturation at pH 7.5. Unlike at pH 5.0, TPCK did not show any additional inactivation of the enzyme at pH 7.5.

In another experiment, the enzyme preparation after ammonium sulfate fractionation was purified through DEAE-Tris acryl column chromatography. The quantitative test for proteolytic activity revealed that this enzyme preparation contained 1 mg/litre equivalent of trypsin-like activity. These trace amounts of proteases were sufficient to cause denaturation of the enzyme. A stability study using various inhibitors of proteases: aprotinin (.5 mg/ml), soybean trypsin inhibitor (5 mg/ml), p-aminobenzamidine (5mM), benzamidine (5mM), α,α′-bipyridal (2 mM) and leupeptin (0.5 mg/ml) was done. The enzyme was stored at 4° C in 50 mM imidazole chloride, 2 mM Mg acetate and .2 mM EDTA, pH 7.5. The activity was assayed at different times. Aprotinin was the most potent stabilizer and the enzyme was stable in its presence for over one month. Leupeptin was equally effective. The enzymes were stable in the presence of soya bean trypsin inhibitor for one week. Enzyme activity was protected for fourteen (14) days in the presence of p-aminobenzamidine and for five (5) days with benzamidine. α,α′-bipyridal was the least effective of the stabilizers tested, but still offered some protection against degradation by proteases.

For suppressing proteolytic activity during the purification of the PAGM/PGM enzyme solution; p-aminobenzamidine as a protease inhibitor was found to be suitable. As large volumes of enzyme solution are prepared in the isolation of these enzymes, use of other protease inhibitors such as soya bean trypsin inhibitor, leupeptin and aprotinin is cost prohibitory. Further, during the final stages of purification of PGM/PAGM, use of proteins as proteolytic inhibitors are inappropriate because protein contaminants are difficult to separate from the enzyme preparation. Since p-Aminobenzamidine is a small molecular weight compound it can be easily removed from the enzyme solution by dialysis or by pressure filtration.

These results demonstrate the clinical utility of the invention.

## Claims

1. A stable aqueous enzyme composition which comprises an enzyme and, as stabilizer, a proteolytic inhibitor.

2. A composition according to claim 1, wherein the enzyme is phosphoenol pyruvate carboxylase.

3. A composition according to claim 2, wherein the stabilizer is phenyl methyl sulfonyl fluoride, α-antitrypsin inhibitor, partially purified ovomucoid, ovoinhibitor or soya bean trypsin inhibitor.

4. A composition according to claim 1, wherein the enzyme is phosphoglucomutase and N-acetyl phosphoglucoseamine mutase.

5. A composition according to claim 4, wherein the stabilizer is soya bean trypsin inhibitor, α-antitrypsin inhibitor, aprotinin, p-aminobenzamidine, benzamidine, leupeptin or α,α′-bipyridal.

6. A method of stabilizing an aqueous enzyme composition which comprises including in the composition a proteolytic inhibitor as a stabilizer.

7. A method according to claim 6, wherein the enzyme is phosphoenol pyruvate carboxylase.

8. A method according to claim 7, wherein the stabilizer is phenyl methyl sulfonyl fluoride, α-antitrypsin inhibitor, partially purified ovomucoid, ovoinhibitor or soya bean trypsin inhibitor.

9. A method according claim 6, wherein the enzyme is phosphoglucomutase and N-acetyl phosphoglucoseamine mutase.

10. A method according to claim 9, wherein the stabilizer is soya bean trypsin inhibitor, antitrypsin inhibitor, aprotinin, p-aminobenzamidine, leupeptin, benzamidine or α,α′-bipyridal.

FIG. 1

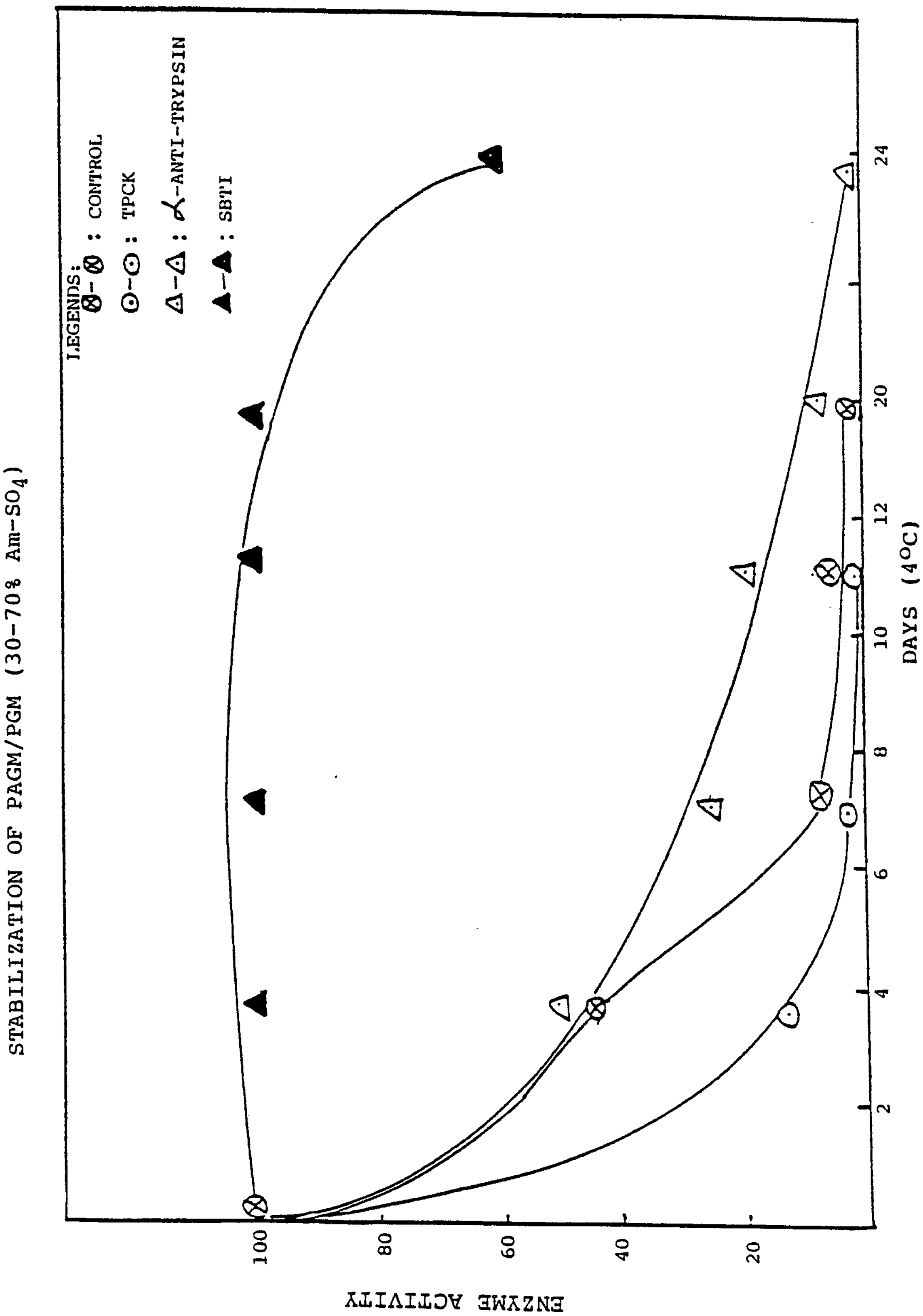
STABILIZATION OF PAGM/PGM (30-70% Am-SO4)
LEGENDS:
⊗-⊗ : CONTROL
⊙-⊙ : TPCK
Δ-Δ : α-ANTI-TRYPSIN
▲-▲ : SBTI
ENZYME ACTIVITY
100
80
60
40
20
DAYS (4°C)
2
4
6
8
10
12
20
24